(19) Europäisches Patentamt
European Patent Office
Office européen des brevets

(11) **EP 3 896 094 A1**

(12) **EUROPEAN PATENT APPLICATION**
published in accordance with Art. 153(4) EPC

(43) Date of publication:
**20.10.2021 Bulletin 2021/42**

(51) Int Cl.:
**C08F 20/06** (2006.01)   **C08J 3/12** (2006.01)
**A61F 13/53** (2006.01)   **B01J 20/26** (2006.01)
**B01J 20/28** (2006.01)

(21) Application number: **19895077.6**

(22) Date of filing: **12.12.2019**

(86) International application number:
**PCT/JP2019/048819**

(87) International publication number:
**WO 2020/122216 (18.06.2020 Gazette 2020/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2018   JP 2018232843**
**22.03.2019   JP 2019054973**

(71) Applicant: **Sumitomo Seika Chemicals Co., Ltd.**
**Kako-gun, Hyogo 675-0145 (JP)**

(72) Inventor: **NISHIDA Moe**
**Himeji-shi, Hyogo 672-8076 (JP)**

(74) Representative: **Nederlandsch Octrooibureau**
**P.O. Box 29720**
**2502 LS The Hague (NL)**

(54) **WATER-ABSORBENT RESIN PARTICLES, ABSORBER, AND ABSORBENT ARTICLE**

(57)    An absorbent article 100 includes an absorber 10, the absorber 10 contains water-absorbent resin particles 10a, and a standard deviation between a water retention amount of 0.01% by mass sodium salt aqueous solution, a water retention amount of 0.01% by mass potassium salt aqueous solution, a water retention amount of 0.01% by mass magnesium salt aqueous solution, and a water retention amount of 0.01% by mass calcium salt aqueous solution in the water-absorbent resin particles 10a is 20 g/g or less.

*Fig.1*

**Description**

**Technical Field**

The present invention relates to water-absorbent resin particles, an absorber, and an absorbent article.

**Background Art**

In the related art, an absorber containing water-absorbent resin particles has been used in an absorbent article for absorbing a liquid containing water such as urine as a main component. For example, Patent Literature 1 below discloses a method for producing water-absorbent resin particles having a particle diameter suitably used for an absorbent article such as a diaper. In addition, Patent Literature 2 discloses a method of using a hydrogel-absorbent polymer having specific saline flow inducibility and performance under pressure, as an effective absorbent member for storing a body fluid such as urine.

**Citation List**

**Patent Literature**

[Patent Literature 1] Japanese Unexamined Patent Publication No. H6-345819
[Patent Literature 2] Japanese Unexamined Patent Publication No. H9-510889

**Summary of Invention**

**Technical Problem**

Usually, an absorber used for an absorbent article is required to absorb various liquids (urine, sweat, and the like) containing metal ions. Here, if the liquid provided to the absorber does not sufficiently permeate the absorber, there may occur a problem that the excess liquid leaks to the outside of the absorber, for example, the excess liquid flows on the surface thereof. Therefore, it is necessary for the liquid containing metal ions to permeate the absorber at a sufficient speed, and it is required that a suitable permeation rate can be stably obtained without depending on the composition of the liquid.

An object of an aspect of the present invention is to provide water-absorbent resin particles that provide an absorber capable of stably obtaining a suitable permeation rate without depending on the composition of the liquid. In addition, an object of another aspect of the present invention is to provide an absorber using the water-absorbent resin particles. In addition, an object of another aspect of the present invention is to provide an absorbent article using the absorber.

**Solution to Problem**

The present inventor has found that, in a case where water-absorbent resin particles in the related art are used, the permeation rate of the liquid into the absorber containing the water-absorbent resin particles depends on the metal ion concentration in the liquid. For example, the metal ion concentration in urine, sweat, and the like can fluctuate due to factors such as individual differences and seasonal differences, and since the permeation rate of the liquid fluctuates due to fluctuations in these factors, in a case where water-absorbent resin particles in the related art are used, there is a case where a desired permeation rate may not be obtained depending on the composition of the liquid (differences between types such as urine and sweat, differences due to composition fluctuations of the same type liquid, and the like). On the other hand, the present inventor found that, by obtaining water-absorbent resin particles having a suitable standard deviation with respect to a water retention amount of an aqueous solution of a specific metal salt in the water-absorbent resin particles, in the absorber using such water-absorbent resin particles, a suitable permeation rate can be stably achieved without depending on the composition of the liquid.

An aspect of the present invention is to provide water-absorbent resin particles, in which a standard deviation between a water retention amount of 0.01% by mass sodium salt aqueous solution, a water retention amount of 0.01% by mass potassium salt aqueous solution, a water retention amount of 0.01% by mass magnesium salt aqueous solution, and a water retention amount of 0.01% by mass calcium salt aqueous solution is 20 g/g or less.

According to the above-mentioned water-absorbent resin particles, an absorber capable of stably obtaining a suitable permeation rate without depending on the composition of the liquid is provided.

Another aspect of the present invention provides an absorber containing the above-mentioned water-absorbent resin particles.

Another aspect of the present invention provides a water-absorbent article including the above-mentioned absorber.

**Advantageous Effects of Invention**

According to an aspect of the present invention, it is possible to provide water-absorbent resin particles that provide an absorber capable of stably obtaining a suitable permeation rate without depending on the composition of the liquid. In addition, according to another aspect of the present invention, it is possible to provide an absorber using the water-absorbent resin particles. In addition, according to another aspect of the present invention, it is possible to provide an absorbent article using the absorber. According to another aspect of the present invention, it is possible to provide use of resin particles, an absorber, and an absorbent article to the absorption of a liquid containing metal ions.

**Brief Description of Drawings**

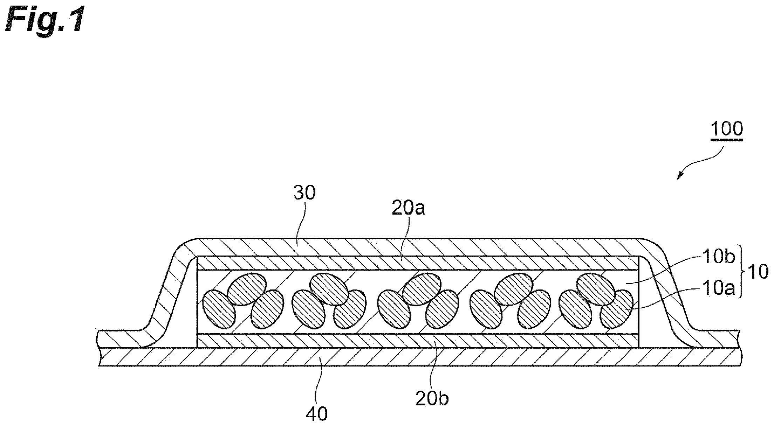

Fig. 1 is a cross-sectional view showing an example of an absorbent article.

**Description of Embodiments**

Hereinafter, embodiments of the present invention will be described in detail. However, the present invention is not limited to the following embodiments, and can be variously modified and implemented within the scope of the gist thereof.

In the present specification, "acrylic" and "methacryl" are collectively referred to as "(meth)acrylic". Similarly, "acrylate" and "methacrylate" are also referred to as "(meth)acrylate". In a numerical value range described in stages in the present specification, an upper limit value or a lower limit value of the numerical value range of a stage can be optionally combined with the upper limit value or the lower limit value of the numerical value range of another stage. In a numerical value range described in the present specification, the upper limit value or the lower limit value of the numerical value range may be replaced with the value shown in the examples. "Water-soluble" means that it exhibits a solubility in water of 5% by mass or more at 25°C. Materials exemplified in the present specification may be used alone, or may be used in combination of two or more. The content of each component in the composition means the total amount of a plurality of substances present in the composition in a case where the plurality of substances corresponding to each component are present in the composition, unless otherwise specified.

In the water-absorbent resin particles of the present embodiment, a standard deviation (hereinafter, referred to as "standard deviation S") between the water retention amount of 0.01% by mass sodium salt aqueous solution, the water retention amount of 0.01% by mass potassium salt aqueous solution, the water retention amount of 0.01% by mass magnesium salt aqueous solution, and the water retention amount of 0.01% by mass calcium salt aqueous solution is 20 g/g or less. That is, when these aqueous solutions are retained in the water-absorbent resin particles of the present embodiment, the water-absorbent resin particles of the present embodiment provides the standard deviation S of 20 g/g or less. The water retention amount that provides the standard deviation S is a water retention amount of the water-absorbent resin particles at the time of drying, and may be a water retention amount at 25°C. The water-absorbent resin particles of the present embodiment are better in absorbency of urine, sweat, blood (for example, menstrual blood), and the like. The water-absorbent resin particles of the present embodiment can be used as a constituent component of the absorber of the present embodiment.

According to the water-absorbent resin particles of the present embodiment, an absorber capable of stably obtaining a suitable permeation rate without depending on the composition of the liquid is provided. The liquid to be absorbed by the water-absorbent resin particles of the present embodiment may contain at least one selected from the group consisting of sodium ions, potassium ions, magnesium ions, and calcium ions. According to the water-absorbent resin particles of the present embodiment, an absorber capable of stably obtaining a suitable permeation rate without depending on the concentration of at least one metal ion selected from the group consisting of sodium ions, potassium ions, magnesium ions, and calcium ions is provided. The liquid to be absorbed by the water-absorbent resin particles of the present embodiment may be an aqueous solution of at least one selected from the group consisting of chloride salt and sulfide salt as a metal salt containing at least one selected from the group consisting of sodium ions, potassium ions, magnesium ions, and calcium ions.

The standard deviation S can be obtained by the following formula (A). In the formula (A), n is 4 (the above-mentioned four kinds of aqueous solutions), $x_i$ represents a water retention amount of each aqueous solution, and $x_a$ represents an average value of the water retention amount of four kinds of aqueous solutions.

[Formula 1]

$$S = \sqrt{\frac{1}{n}\sum_{i=1}^{n}(x_i - x_a)^2} \quad \cdots (A)$$

The standard deviation S is preferably 18 g/g or less, more preferably 16 g/g or less, further more preferably 14 g/g or less, particularly preferably 13 g/g or less, extremely preferably 12 g/g or less, and extraordinarily preferably 11 g/g or less, from a viewpoint of easily stably obtaining a suitable permeation rate in a case of being used for an absorber. The standard deviation S is 0 g/g or more, may exceed 0 g/g, may be 5 g/g or more, may be 8 g/g or more, and may be 10 g/g or more.

The water-absorbent resin particles of the present embodiment may be any water-absorbent resin particles as long as the water-absorbent resin particles can retain water, and the liquid to be absorbed can contain water. The water-absorbent resin particles of the present embodiment are water-absorbent resin particles capable of water-retaining at least one selected from the group consisting of a sodium salt aqueous solution, a potassium salt aqueous solution, a magnesium salt aqueous solution, and a calcium salt aqueous solution, and are, for example, water-absorbent resin particles capable of water-retaining at least one selected from the group consisting of a sodium chloride aqueous solution, a potassium chloride aqueous solution, a magnesium chloride, and a calcium chloride aqueous solution. In the water-absorbent resin particles of the present embodiment, for example, the standard deviation between the water retention amount of 0.01% by mass sodium chloride aqueous solution, the water retention amount of 0.01% by mass potassium chloride aqueous solution, the water retention amount of 0.01% by mass magnesium chloride aqueous solution, and the water retention amount of 0.01% by mass calcium chloride aqueous solution is 20 g/g or less.

In the water-absorbent resin particles of the present embodiment, at least one selected from the group consisting of the water retention amount of 0.01% by mass sodium salt aqueous solution, the water retention amount of 0.01% by mass potassium salt aqueous solution, the water retention amount of 0.01% by mass magnesium salt aqueous solution, and the water retention amount of 0.01% by mass calcium salt aqueous solution is preferably in the following range (for example, 150 g/g or more).

The water retention amount of 0.01% by mass sodium salt aqueous solution is preferably 50 g/g or more, more preferably 100 g/g or more, further more preferably 150 g/g or more, particularly preferably 200 g/g or more, and extremely preferably 250 g/g or more, from a viewpoint of easily stably obtaining a suitable permeation rate in a case of being used for an absorber. The upper limit of the water retention amount of 0.01% by mass sodium salt aqueous solution is 500 g/g or less, for example.

The water retention amount of 0.01% by mass potassium salt aqueous solution is preferably 50 g/g or more, more preferably 100 g/g or more, further more preferably 150 g/g or more, particularly preferably 200 g/g or more, and extremely preferably 250 g/g or more, from a viewpoint of easily stably obtaining a suitable permeation rate in a case of being used for an absorber. The upper limit of the water retention amount of 0.01% by mass potassium salt aqueous solution is 500 g/g or less, for example.

The water retention amount of 0.01% by mass magnesium salt aqueous solution is preferably 50 g/g or more, more preferably 100 g/g or more, further more preferably 150 g/g or more, particularly preferably 200 g/g or more, extremely preferably 220 g/g or more, and extraordinarily preferably 250 g/g or more, from a viewpoint of easily stably obtaining a suitable permeation rate in a case of being used for an absorber. The upper limit of the water retention amount of 0.01 mass% magnesium salt aqueous solution is 500 g/g or less, for example.

The water retention amount of 0.01% by mass calcium salt aqueous solution is preferably 50 g/g or more, more preferably 100 g/g or more, further more preferably 150 g/g or more, particularly preferably 180 g/g or more, extremely preferably 200 g/g or more, and extraordinarily preferably 240 g/g or more, from a viewpoint of easily stably obtaining a suitable permeation rate in a case of being used for an absorber. The upper limit of the water retention amount of 0.01 mass% calcium salt aqueous solution is 500 g /g or less, for example.

The water-absorbent resin particles of the present embodiment can have a high water-absorbing ability with respect to physiological saline. The water retention amount of physiological saline of the water-absorbent resin particles of the present embodiment is preferably 20 g/g or more, 25 g/g or more, 27 g/g or more, 30 g/g or more, 32 g/g or more, 35 g/g or more, 37 g/g or more, 39 g/g or more, or 40 g/g or more, from a viewpoint of easily suitably enhancing absorption capacity of the absorber. The water retention amount of physiological saline of the water-absorbent resin particles may be 70 g/g or less, 65 g/g or less, 60 g/g or less, 55 g/g or less, 50 g/g or less, 48 g/g or less, or 45 g/g or less. The water retention amount of physiological saline of the water-absorbent resin particles may be 20 to 70 g/g, 25 to 65 g/g, 27 to 60 g/g, 30 to 57 g/g, 32 to 55 g/g, 30 to 70 g/g, 32 to 70 g/g, 35 to 70 g/g, 38 to 65 g/g, 40 to 65 g/g, 40 to 60 g/g, or 40 to 55 g/g. As the water retention amount of physiological saline of the water-absorbent resin particles, a water retention amount at 25°C can be used. The water retention amount of physiological saline of the water-absorbent resin particles can be measured by the method described in International Publication No. 2018/181565.

Examples of the shape of the water-absorbent resin particles of the present embodiment include substantially spherical, crushed, and granular shapes. The medium particle diameter of the water-absorbent resin particles of the present embodiment may be 250 to 850 μm, 300 to 700 μm, or 300 to 600 μm. The water-absorbent resin particles of the present embodiment may have a desired particle size distribution at the time of being obtained by a production method to be described later, but the particle size distribution may be adjusted by performing an operation such as particle size adjustment using classification with a sieve.

The water-absorbent resin particles of the present embodiment can contain a crosslinking polymer obtained by polymerizing a monomer containing an ethylenically unsaturated monomer (crosslinking polymer having a structural unit derived from the ethylenically unsaturated monomer), for example. That is, the water-absorbent resin particles of the present embodiment can have a structural unit derived from an ethylenically unsaturated monomer. Examples of the polymerization method include a reverse phase suspension polymerization method, an aqueous solution polymerization method, a bulk polymerization method, and a precipitation polymerization method. Among these, the reverse phase suspension polymerization method or the aqueous solution polymerization method is preferable from a viewpoint of ensuring good water-absorbent characteristics of the obtained water-absorbent resin particles and facilitating control of the polymerization reaction. In the following, as a method for polymerizing an ethylenically unsaturated monomer, a reverse phase suspension polymerization method will be described as an example.

The ethylenically unsaturated monomer is preferably water-soluble, and examples thereof include (meth)acrylic acid and a salt thereof, 2-(meth)acrylamide-2-methylpropanesulfonic acid and a salt thereof, (meth)acrylamide, N, N-dimethyl (meth)acrylamide, 2-hydroxyethyl (meth)acrylate, N-methylol (meth)acrylamide, polyethylene glycol mono(meth)acrylate, N, N-diethylaminoethyl (meth)acrylate, N, N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone, or may be used in combination of two or more. Functional groups such as a carboxyl group and an amino group of the above-mentioned monomers can function as functional groups capable of crosslinking in a surface crosslinking step to be described later.

Among these, from a viewpoint of industrial availability, the ethylenically unsaturated monomer preferably contains at least one compound selected from the group consisting of (meth)acrylic acid and a salt thereof, acrylamide, methacrylamide, and N, N-dimethylacrylamide, and more preferably contains at least one compound selected from the group consisting of (meth)acrylic acid and a salt thereof, and acrylamide. From a viewpoint of further enhancing the water-absorbent characteristics, the ethylenically unsaturated monomer further more preferably contains at least one compound selected from the group consisting of (meth)acrylic acid and a salt thereof.

As the monomer for obtaining the water-absorbent resin particles, a monomer other than the above-mentioned ethylenically unsaturated monomer may be used. Such a monomer can be used by being mixed with an aqueous solution containing the above-mentioned ethylenically unsaturated monomer, for example. The use amount of the ethylenically unsaturated monomer is preferably 70 to 100 mol% with respect to a total amount of the monomer (the total amount of the monomer for obtaining the water-absorbent resin particles. For example, a total amount of the monomers that provide a structural unit of the crosslinking polymer. The same applies hereinafter). Among these, the ratio of (meth)acrylic acid and a salt thereof is more preferably 70 to 100 mol% with respect to the total amount of the monomers. "Ratio of (meth)acrylic acid and a salt thereof" means the ratio of the total amount of (meth)acrylic acid and a salt thereof.

According to the present embodiment, as an example of the water-absorbent resin particles, it is possible to provide water-absorbent resin particles containing a crosslinking polymer having a structural unit derived from the ethylenically unsaturated monomer, in which the ethylenically unsaturated monomer contains at least one compound selected from the group consisting of (meth)acrylic acid and a salt thereof, the ratio of (meth)acrylic acid and a salt thereof is 70 to 100 mol% with respect to the total amount of the monomer for obtaining the water-absorbent resin particles (for example, the total amount of the monomer that provides a structural unit of the crosslinking polymer), and the standard deviation between a water retention amount of 0.01% by mass sodium salt aqueous solution, a water retention amount of 0.01% by mass potassium salt aqueous solution, a water retention amount of 0.01% by mass magnesium salt aqueous solution, and a water retention amount of 0.01% by mass calcium salt aqueous solution is 20 g/g or less.

The ethylenically unsaturated monomer is usually preferably used as an aqueous solution. The concentration of the ethylenically unsaturated monomer in the aqueous solution containing the ethylenically unsaturated monomer (hereinafter, simply referred to as "monomer aqueous solution") is preferably 20% by mass or more and a saturated concentration or less, more preferably 25 to 70% by mass, and further more preferably 30 to 55% by mass. Examples of the water used in the aqueous solution include tap water, distilled water, and ion-exchanged water.

In a case where the ethylenically unsaturated monomer has an acid group, the monomer aqueous solution may be used by neutralizing the acid group with an alkaline neutralizing agent. The degree of neutralization of the ethylenically unsaturated monomer by the alkaline neutralizing agent is preferably 10 to 100 mol%, more preferably 50 to 90 mol%, and further more preferably 60 to 80 mol% of the acid group in the ethylenically unsaturated monomer, from a viewpoint of increasing an osmotic pressure of the obtained water-absorbent resin particles, and further increasing the water-absorbent characteristics (water retention amount and the like). Examples of the alkaline neutralizing agent include alkali metal salts such as sodium hydroxide, sodium carbonate, sodium hydrogen carbonate, potassium hydroxide, and potassium carbonate; and ammonia. The alkaline neutralizing agent may be used alone, or may be used in combination of two or more. The alkaline neutralizing agent may be used in the form of an aqueous solution to simplify the neutralization operation. Neutralization of the acid group of the ethylenically unsaturated monomer can be performed by adding an aqueous solution of sodium hydroxide, potassium hydroxide, or the like dropwise in the above-mentioned monomer aqueous solution and mixing therewith.

In a reverse phase suspension polymerization method, a monomer aqueous solution is dispersed in a hydrocarbon dispersion medium in the presence of a surfactant, and polymerization of the ethylenically unsaturated monomer can be performed using a radical polymerization initiator or the like. As the radical polymerization initiator, a water-soluble radical polymerization initiator can be used.

Examples of the surfactant include a nonionic surfactant, and an anionic surfactant. Examples of the nonionic surfactant include sorbitan fatty acid ester, (poly)glycerin fatty acid ester ("(poly)" means both of a case where there is a prefix of "poly" and a case where there is no prefix thereof. The same applies hereinafter), sucrose fatty acid ester, polyoxyethylene sorbitan fatty acid ester, polyoxyethylene glycerin fatty acid ester, sorbitol fatty acid ester, polyoxyethylene sorbitol fatty acid ester, polyoxyethylene alkyl ether, polyoxyethylene alkyl phenyl ether, polyoxyethylene castor oil, polyoxyethylene hydrogenated castor oil, alkylallyl formaldehyde condensed polyoxyethylene ether, polyoxyethylene polyoxypropylene block copolymer, polyoxyethylene polyoxypropyl alkyl ether, and polyethylene glycol fatty acid ester. Examples of the anionic surfactant include fatty acid salt, alkylbenzene sulfonate, alkylmethyl taurate, polyoxyethylene alkylphenyl ether sulfate, polyoxyethylene alkyl ether sulfonate, phosphate ester of polyoxyethylene alkyl ether, and phosphate ester of polyoxyethylene alkylallyl ether. The surfactant may be used alone, or may be used in combination of two or more.

From a viewpoint of a good state of the W/O type reverse phase suspension, easily obtaining water-absorbent resin particles having a suitable particle diameter, and industrial availability, the surfactant preferably contains at least one compound selected from the group consisting of a sorbitan fatty acid ester, a polyglycerin fatty acid ester and a sucrose fatty acid ester. From a viewpoint of easily improving the water-absorbent characteristics of the obtained water-absorbent resin particles, the surfactant preferably contains a sucrose fatty acid ester, and more preferably contains a sucrose stearic acid ester.

The use amount of the surfactant is preferably 0.05 to 10 parts by mass, more preferably 0.08 to 5 parts by mass, and further more preferably 0.1 to 3 parts by mass with respect to 100 parts by mass of the monomer aqueous solution from a viewpoint of obtaining a sufficient effect on the use amount and economic efficiency.

In the reverse phase suspension polymerization, a polymeric dispersant may be used in combination with the above-mentioned surfactant. Examples of the polymeric dispersant include maleic anhydride-modified polyethylene, maleic anhydride-modified polypropylene, maleic anhydride-modified ethylene/propylene copolymer, maleic anhydride-modified EPDM (ethylene propylene diene terpolymer), maleic anhydride-modified polybutadiene, maleic anhydride/ethylene copolymer, maleic anhydride/propylene copolymer, maleic anhydride/ethylene/propylene copolymer, maleic anhydride/butadiene copolymer, polyethylene, polypropylene, ethylene/propylene copolymer, oxidized polyethylene, oxidized polypropylene, oxidized ethylene/propylene copolymer, ethylene/acrylic acid copolymer, ethyl cellulose, and ethyl hydroxyethyl cellulose. The polymeric dispersant may be used alone or may be used in combination of two or more. From a viewpoint of better dispersion stability of the monomer, the polymeric dispersant is preferably at least one selected from the group consisting of maleic anhydride-modified polyethylene, maleic anhydride-modified polypropylene, maleic anhydride-modified ethylene/propylene copolymer, maleic anhydride/ethylene copolymer, maleic anhydride/propylene copolymer, maleic anhydride/ethylene/propylene copolymer, polyethylene, polypropylene, ethylene/propylene copolymer, oxidized polyethylene, oxidized polypropylene, and oxidized ethylene/propylene copolymer.

The use amount of the polymeric dispersant is preferably 0.05 to 10 parts by mass, more preferably 0.08 to 5 parts by mass, and further more preferably 0.1 to 3 parts by mass with respect to 100 parts by mass of the monomer aqueous solution, from a viewpoint of obtaining a sufficient effect on the use amount and economic efficiency.

The hydrocarbon dispersion medium may contain at least one compound selected from the group consisting of chain aliphatic hydrocarbons having 6 to 8 carbon atoms and alicyclic hydrocarbons having 6 to 8 carbon atoms. Examples of the hydrocarbon dispersion medium include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone, or may be used in combination of two or more.

The hydrocarbon dispersion medium may contain at least one selected from the group consisting of n-heptane and cyclohexane from a viewpoint of industrial availability and stable quality. In addition, from the same viewpoint, as the mixture of the above-mentioned hydrocarbon dispersion medium, for example, commercially available Exxsol Heptane (manufactured by ExxonMobil: containing 75% to 85% of n-heptane and isomeric hydrocarbons) may be used.

The use amount of the hydrocarbon dispersion medium is preferably 30 to 1000 parts by mass, more preferably 40 to 500 parts by mass, and further more preferably 50 to 300 parts by mass with respect to 100 parts by mass of the monomer aqueous solution, from a viewpoint of appropriately removing the heat of polymerization and easily controlling the polymerization temperature. In a case where the use amount of the hydrocarbon dispersion medium is 30 parts by mass or more, the polymerization temperature tends to be easily controlled. In a case where the use amount of the hydrocarbon dispersion medium is 1000 parts by mass or less, the productivity of polymerization tends to be improved, which is economical.

The radical polymerization initiator is preferably water-soluble, and examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumylperoxide, t-butylperoxyacetate, t-butylperoxyisobutyrate, t-butylperoxypivalate, and hydrogen peroxide; azo compounds such as 2,2'-azobis(2-amidinopropane) dihydrochloride, 2,2'-azobis [2-(N-phenylamidino) propane] dihydrochloride, 2,2'-azobis [2-(N-allylamidino) propane] dihydrochloride, 2,2'-azobis [2-(2-imidazoline-2-yl) propane] dihydrochloride, 2,2'-azobis {2-[1-(2-hydroxyethyl) -2-imidazoline-2-yl] propane} dihydrochloride, 2,2'-azobis {2-methyl-N-[1,1-bis (hydroxymethyl)-2-hydroxyethyl] propionamide}, 2,2'-azobis [2-methyl-N-(2-hydroxyethyl)-propionamide], and 4,4'-azobis (4-cyanovaleric acid). The radical polymerization initiator may be used alone, or may be used in combination of two or more. The radical polymerization initiator is preferably at least one selected from the group consisting of potassium persulfate, ammonium persulfate, sodium persulfate, 2,2'-azobis (2-amidinopropane) dihydrochloride, 2,2'-azobis [2- (2-imidazoline-2-yl) propane] dihydrochloride, and 2,2'-azobis {2-[1-(2-hydroxyethyl)-2-imidazoline-2-yl] propane} dihydrochloride.

The use amount of the radical polymerization initiator may be 0.00005 to 0.01 mol with respect to 1 mol of the ethylenically unsaturated monomer. In a case where the use amount of the radical polymerization initiator is 0.00005 mol or more, the polymerization reaction does not require a long time and is efficient. In a case where the use amount of the radical polymerization initiator is 0.01 mol or less, the occurrence of a rapid polymerization reaction is easily inhibited.

The above-mentioned radical polymerization initiator can also be used as a redox polymerization initiator in combination with a reducing agent such as sodium sulfite, sodium hydrogen sulfite, ferrous sulfate, and L-ascorbic acid.

At the time of the polymerization reaction, the monomer aqueous solution used for the polymerization may contain a chain transfer agent. Examples of the chain transfer agent include hypophosphites, thiols, thiolic acids, secondary alcohols, and amines.

The monomer aqueous solution used for the polymerization may contain a thickener in order to control the particle diameter of the water-absorbent resin particles. Examples of the thickener include hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, and polyethylene oxide. In a case where the stirring speed at the time of polymerization is the same, the higher the viscosity of the monomer aqueous solution, the larger the medium particle diameter of the obtained particles tends to be.

Crosslinking by self-crosslinking may occur during polymerization, but crosslinking may be further performed by using an internal crosslinking agent. In a case where an internal crosslinking agent is used, the water-absorbent characteristics of the water-absorbent resin particles are easily controlled. The internal crosslinking agent is usually added to a reaction solution during the polymerization reaction. Examples of the internal crosslinking agent include di or tri (meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the above-mentioned polyols with unsaturated acids (such as maleic acid and fumaric acid); bis (meth)acrylamides such as N, N'-methylene bis (meth)acrylamide; di or tri (meth)acrylic acid esters obtained by reacting polyepoxide with (meth)acrylic acid; di (meth)acrylic acid carbamil esters obtained by reacting polyisocyanate (such as tolylene diisocyanate and hexamethylene diisocyanate) with hydroxyethyl (meth)acrylate; compounds having two or more polymerizable unsaturated groups such as allylated starch, allylated cellulose, diallyl phthalate, N, N', N"-triallyl isocyanurate, and divinylbenzene; polyglycidyl compounds such as (poly)ethylene glycol diglycidyl ether, (poly) propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromhydrin, and $\alpha$-methylepichlorohydrin; and compounds having two or more reactive functional groups such as isocyanate compounds (2,4-tolylene diisocyanate and hexamethylene diisocyanate). The internal crosslinking agent may be used alone, or may be used in combination of two or more. The internal crosslinking agent is preferably a polyglycidyl compound, more preferably a diglycidyl ether compound, and further more preferably at least one selected from the group consisting of (poly)ethylene glycol diglycidyl ether, (poly)propylene glycol diglycidyl ether, and (poly)glycerin diglycidyl ether.

The use amount of the internal crosslinking agent is preferably 0 to 0.03 mol, more preferably 0.00001 to 0.01 mol, and further more preferably 0.00002 to 0.005 mol per 1 mol of the ethylenically unsaturated monomer from a viewpoint of suppressing water-soluble property by appropriately crosslinking the obtained polymer to easily obtain the sufficient water absorption amount.

It is possible to perform heating while stirring in a state of mixing an ethylenically unsaturated monomer, a radical polymerization initiator, a surfactant, a polymeric dispersant, a hydrocarbon dispersion medium, or the like (if necessary, additionally an internal crosslinking agent), and to perform reverse phase suspension polymerization in a water-in-oil system.

When performing the reverse phase suspension polymerization, a monomer aqueous solution containing an ethylenically unsaturated monomer is dispersed in a hydrocarbon dispersion medium in the presence of a surfactant (if necessary, additionally a polymeric dispersant). At this time, before the start of the polymerization reaction, the timing of adding the surfactant, the polymeric dispersant, or the like may be either before or after the addition of the monomer aqueous solution.

Among these, from a viewpoint of easily reducing the amount of the hydrocarbon dispersion medium remaining in the obtained water-absorbent resin, it is preferable to perform polymerization after dispersing the monomer aqueous solution in the hydrocarbon dispersion medium in which the polymeric dispersant is dispersed and then further dispersing the surfactant.

Reverse phase suspension polymerization can be performed in one stage, or in multiple stages of two or more stages. Reverse phase suspension polymerization is preferably performed in two to three stages from a viewpoint of increasing productivity.

In a case where reverse phase suspension polymerization is performed in multiple stages of two or more stages, a first stage reverse phase suspension polymerization is performed, an ethylenically unsaturated monomer is added to the reaction mixture obtained in the first polymerization reaction and mixed therewith, and second and subsequent stages of reverse phase suspension polymerization may be performed in the same method as the first stage. In the reverse phase suspension polymerization in each stage of the second and subsequent stages, in addition to the ethylenically unsaturated monomer, the above-mentioned radical polymerization initiator and/or internal crosslinking agent is preferably added in a range of a molar ratio of each component with respect to the above-mentioned ethylenically unsaturated monomer, based on an amount of the ethylenically unsaturated monomer added at the time of the second and subsequent stages of reverse phase suspension polymerization, to perform reverse phase suspension polymerization. In the reverse phase suspension polymerization in each stage of second and subsequent stages, an internal crosslinking agent may be used if necessary. In a case of using the internal crosslinking agent, the internal crosslinking agent is preferably added within a range of the molar ratio of each component with respect to the above-mentioned ethylenically unsaturated monomer based on the amount of the ethylenically unsaturated monomer provided in each stage, to perform reverse phase suspension polymerization.

The temperature of the polymerization reaction varies depending on the used radical polymerization initiator, and the temperature is preferably 20°C to 150°C, and more preferably 40°C to 120°C from a viewpoint of rapidly proceeding the polymerization and shortening the polymerization time to enhance economic efficiency, and easily removing polymerization heat and smoothly performing reaction. The reaction time is usually 0.5 to 4 hours. The completion of the polymerization reaction can be confirmed by stopping the temperature rise in the reaction system. Thus, the polymer of the ethylenically unsaturated monomer is usually obtained in a state of a hydrogel.

After the polymerization, a crosslinking agent may be added to the obtained hydrogel-like polymer and heated to perform post-polymerization crosslinking. By performing post-polymerization crosslinking, a degree of crosslinking of the hydrogel-like polymer can be increased, and the water-absorbent characteristics can be further improved.

Examples of the crosslinking agent for performing post-polymerization crosslinking include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups such as (poly)ethylene glycol diglycidyl ether, (poly)propylene glycol diglycidyl ether, and (poly)glycerin diglycidyl ether; haloepoxy compounds such as epichlorohydrin, epibromhydrin, and $\alpha$-methylepichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis [N, N-di ($\beta$-hydroxyethyl)] adipamide. Among these, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. The crosslinking agent may be used alone, or may be used in combination of two or more.

The amount of the crosslinking agent used for post-polymerization crosslinking is preferably 0 to 0.03 mol, more preferably 0 to 0.01 mol, and further more preferably 0.00001 to 0.005 mol per 1 mol of a water-soluble ethylenically unsaturated monomer from a viewpoint of exhibiting suitable water-absorbent characteristics by appropriately crosslinking the obtained hydrogel-like polymer. In a case where the addition amount of the crosslinking agent is within the above-mentioned range, water-absorbent resin particles that provide an absorber capable of stably obtaining a suitable permeation rate without depending on the composition of the liquid are easily obtained.

The timing of adding the crosslinking agent used for post-polymerization crosslinking may be after the polymerization of the ethylenically unsaturated monomer used for the polymerization, and in the case of multiple-stage polymerization, it is preferable to add the crosslinking agent after the multiple-stage polymerization. Considering fluctuation in water due to heat generation at the time of polymerization and after polymerization, retention due to process delay, opening of the system at the time of addition of the crosslinking agent, addition of water due to the addition of the crosslinking agent, or the like, the crosslinking agent for post-polymerization crosslinking is preferably added in a region of [water content (immediately after polymerization) ± 3% by mass] from a viewpoint of water content (to be described later).

Subsequently, the polymer particles (for example, polymer particles having a structural unit derived from an ethylenically unsaturated monomer) are obtained by drying in order to remove water from the obtained hydrogel-like polymer. Examples of a drying method include (a) a method of removing water by performing azeotropic distillation by heating from outside in a state where a hydrogel-like polymer is dispersed in a hydrocarbon dispersion medium, and refluxing the hydrocarbon dispersion medium, (b) a method of taking out a hydrogel-like polymer by decantation and drying under reduced pressure, and (c) a method of filtering the hydrogel-like polymer with a filter and drying under reduced pressure. Among these, it is preferable to use the method (a) due to the simplicity in the production process.

It is possible to adjust the particle diameter of water-absorbent resin particles by adjusting a rotation speed of a stirrer during the polymerization reaction, or by adding a flocculant into the system after the polymerization reaction or in the initial stage of drying. By adding a flocculant, it is possible to increase the particle diameter of the obtained water-absorbent resin particles. As the flocculant, an inorganic flocculant can be used. Examples of the inorganic flocculant (for example, powdered inorganic flocculant) include silica, zeolite, bentonite, aluminum oxide, talc, titanium dioxide, kaolin, clay, and hydrotalcite. From a viewpoint of better flocculation effect, the flocculant is preferably at least one selected from the group consisting of silica, aluminum oxide, talc, and kaolin.

In the reverse phase suspension polymerization, a method of adding the flocculant is preferably a method of preliminarily dispersing a flocculant in a hydrocarbon dispersion medium or water of the same type as that used in the polymerization, and then mixing into a hydrocarbon dispersion medium containing a hydrogel-like polymer under stirring.

The addition amount of the flocculant is preferably 0.001 to 1 part by mass, more preferably 0.005 to 0.5 part by mass, and further more preferably 0.01 to 0.2 parts by mass with respect to 100 parts by mass of the ethylenically unsaturated monomer used for the polymerization. In a case where the addition amount of the flocculant is within the above-mentioned range, water-absorbent resin particles having a target particle size distribution can be easily obtained.

In the production of the water-absorbent resin particles, it is preferable to perform crosslinking (surface crosslinking) of a surface portion of a hydrogel-like polymer using a crosslinking agent in a drying step or any subsequent steps. By performing surface crosslinking, the water-absorbent characteristics of the water-absorbent resin particles is easily controlled. The surface crosslinking is preferably performed at the timing when the hydrogel-like polymer has a specific water content. The timing of surface crosslinking is preferably when the water content of the hydrogel-like polymer is 5% to 50% by mass, more preferably when the water content of the hydrogel-like polymer is 10% to 40% by mass, and further more preferably when the water content of the hydrogel-like polymer is 15% to 35% by mass. The water content (mass%) of the hydrogel-like polymer is calculated by the following formula.

$$\text{Water content} = [\text{Ww}/(\text{Ww} + \text{Ws})] \times 100$$

Ww: Water amount of a hydrogel-like polymer obtained by adding water amount used if necessary when mixing a flocculant, a surface crosslinking agent, or the like to an amount obtained by subtracting water amount discharged to the outside of the system in the drying step, from water amount contained in a monomer aqueous solution before polymerization in the entire polymerization step.

Ws: Solid content calculated from the charged amount of materials such as ethylenically unsaturated monomer, crosslinking agent, and initiator that constitute a hydrogel-like polymer.

Examples of the crosslinking agent (surface crosslinking agent) for performing surface crosslinking include compounds having two or more reactive functional groups. Examples of the crosslinking agent include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylpropane triglycidyl ether (poly)propylene glycol polyglycidyl ether, and (poly)glycerol polyglycidyl ether; haloepoxy compounds such as epichlorohydrin, epibromhydrin, and $\alpha$-methylepichlorohydrin; isocyanate compounds such as 2,4-tolylene diisocyanate and hexamethylenediisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis [N, N-di (β-hydroxyethyl)] adipamide. The crosslinking agent may be used alone, or may be used in combination of two or more. The crosslinking agent is preferably a polyglycidyl compound, and more preferably at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

The use amount of the surface crosslinking agent is usually preferably 0.00001 to 0.02 mol, more preferably 0.00005 to 0.01 mol, and further more preferably 0.0001 to 0.005 mol with respect to 1 mol of the ethylenically unsaturated monomer used for polymerization from a viewpoint of easily obtaining suitable water-absorbent characteristics (water retention amount and the like). In a case where the addition amount of the surface crosslinking agent is within the above-mentioned range, water-absorbent resin particles that provide an absorber capable of stably obtaining a suitable permeation rate without depending on the composition of the liquid are easily obtained.

After surface crosslinking, it is possible to obtain polymer particles which are surface-crosslinked dried products by distilling water and a hydrocarbon dispersion medium with a known method.

The water-absorbent resin particles of the present embodiment can further contain additional components such as a gel stabilizer, a metal chelating agent (for example, diethylenetriamine pentasodium pentaacetate), and a flowablility improver (lubricant). Additional components can be disposed inside the polymer particles, on the surface of the polymer particles, or both thereof.

The water-absorbent resin particles may contain a plurality of inorganic particles disposed on the surface of the polymer particles. For example, by mixing the polymer particles and the inorganic particles, it is possible to dispose the inorganic particles on the surface of the polymer particles. The inorganic particles may be silica particles such as amorphous silica.

In a case where the water-absorbent resin particles include inorganic particles disposed on the surface of the polymer particles, the content of the inorganic particles may be in the following range based on the total mass of the polymer particles. The content of the inorganic particles may be 0.05% by mass or more, 0.1% by mass or more, 0.15% by mass or more, or 0.2% by mass or more. The content of the inorganic particles may be 5.0% by mass or less, 3.0% by mass or less, 1.0% by mass or less, 0.5% by mass or less, or 0.3% by mass or less.

The inorganic particles here usually have a minute size as compared with the size of the polymer particles. For example, the average particle diameter of the inorganic particles may be 0.1 to 50 $\mu$m, 0.5 to 30 $\mu$m, or 1 to 20 $\mu$m. The average particle diameter can be measured by a dynamic light scattering method or a laser diffraction/scattering method.

The absorber of the present embodiment contains the water-absorbent resin particles of the present embodiment. The absorber of the present embodiment may contain a fibrous substance, for example, is a mixture containing water-absorbent resin particles and the fibrous substance. For example, the structure of the absorber may be a structure in which the water-absorbent resin particles and the fibrous substance are uniformly mixed, may be a structure in which the water-absorbent resin particles are sandwiched between the fibrous substances formed in the form of a sheet or a layer, or may be other structures.

Examples of the fibrous substance include finely pulverized wood pulp; cotton; cotton linter; rayon; cellulosic fibers such as cellulose acetate; synthetic fibers such as polyamide, polyester and polyolefin; and a mixture of these fibers. The fibrous substance may be used alone, or may be used in combination of two or more. As the fibrous substance, hydrophilic fibers can be used.

The mass ratio of the water-absorbent resin particles in the absorber may be 2% to 100% by mass, 10% to 80% by mass, or 20% to 60% by mass with respect to the total of the water-absorbent resin particles and the fibrous substance.

The content of the water-absorbent resin particles in the absorber is preferably 100 to 1000 g, more preferably 150 to 800 g, and further more preferably 200 to 700 g per 1 m$^2$ of the absorber from a viewpoint of easily obtaining sufficient water absorption performance. The content of the fibrous substance in the absorber is preferably 50 to 800 g, more preferably 100 to 600 g, and further more preferably 150 to 500 g per 1 m$^2$ of the absorber from a viewpoint of easily obtaining sufficient water absorption performance.

In order to enhance the morphological retention before and during use of the absorber, the fibers may be adhered to each other by adding an adhesive binder to the fibrous substance. Examples of the adhesive binder include thermal bonding synthetic fibers, hot melt adhesives, and adhesive emulsions. The adhesive binder may be used alone, or may be used in combination of two or more.

Examples of the thermal bonding synthetic fiber include a total fusion type binder such as polyethylene, polypropylene, and an ethylene-propylene copolymer; and a non-total fusion type binder made of a side-by-side or core-sheath structure of polypropylene and polyethylene. In the above-mentioned non-total fusion type binder, only the polyethylene portion can be thermal-bonded.

Examples of the hot melt adhesive include a mixture of a base polymer such as ethylene-vinyl acetate copolymer, styrene-isoprene-styrene block copolymer, styrene-butadiene-styrene block copolymer, styrene-ethylene-butylene-styrene block copolymer, styrene-ethylene-propylene-styrene block copolymer, and amorphous polypropylene with a tackifier, a plasticizer, an antioxidant, or the like.

Examples of the adhesive emulsion include a polymerization product of at least one monomer selected from the group consisting of methyl methacrylate, styrene, acrylonitrile, 2-ethylhexyl acrylate, butyl acrylate, butadiene, ethylene, and vinyl acetate.

The absorber of the present embodiment may contain an inorganic powder (for example, amorphous silica), a deodorant, an antibacterial agent, a fragrance, or the like. In a case where the water-absorbent resin particles contain inorganic particles, the absorber may contain an inorganic powder in addition to the inorganic particles of the water-absorbent resin particles.

The shape of the absorber of the present embodiment may be a sheet shape, for example. The thickness of the absorber (for example, thickness of the sheet shape absorber) may be 0.1 to 20 mm or 0.3 to 15 mm, for example.

The absorbent article of the present embodiment includes an absorber of the present embodiment. Examples of the absorbent article of the present embodiment include a core wrap that retains an absorber; a liquid permeable sheet disposed on the outermost part at the side where the liquid to be absorbed enters; and a liquid impermeable sheet disposed on the outermost part at the opposite side to the side where the liquid to be absorbed enters. Examples of the absorbent article include diapers (for example, paper diapers), toilet training pants, incontinence pads, sanitary materials (sanitary napkins, tampons, and the like), sweat pads, pet sheets, portable toilet members, and animal excrement treatment materials.

Fig. 1 is a cross-sectional view showing an example of an absorbent article. An absorbent article 100 shown in Fig. 1 includes an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown so that there is a gap between the members, but the members may be in close contact with each other without the gap.

The absorber 10 has a water-absorbent resin particle 10a of the present embodiment and a fiber layer 10b containing a fibrous substance. The water-absorbent resin particles 10a are dispersed in the fiber layer 10b.

The core wrap 20a is disposed on one surface side of the absorber 10 (upper side of the absorber 10 in Fig. 1) in a state of being in contact with the absorber 10. The core wrap 20b is disposed on the other surface side of the absorber 10 (lower side of the absorber 10 in Fig. 1) in a state of being in contact with the absorber 10. The absorber 10 is disposed between the core wrap 20a and the core wrap 20b. Examples of the core wraps 20a and 20b include tissues and non-woven fabrics. The core wrap 20a and the core wrap 20b have a main surface having the same size as that of the absorber 10, for example.

The liquid permeable sheet 30 is disposed on the outermost part at the side where the liquid to be absorbed enters. The liquid permeable sheet 30 is disposed on the core wrap 20a in a state of being in contact with the core wrap 20a. Examples of the liquid permeable sheet 30 include a non-woven fabric made of a synthetic resin such as polyethylene, polypropylene, polyester, and polyamide, and a porous sheet. The liquid impermeable sheet 40 is disposed on the outermost part at the opposite side to the liquid permeable sheet 30 in the absorbent article 100. The liquid impermeable sheet 40 is disposed on a lower side of the core wrap 20b in a state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. The liquid permeable sheet 30 and the liquid impermeable sheet 40 have a main surface wider than the main surface of the absorber 10, and outer edges of the liquid permeable sheet 30 and the liquid impermeable sheet 40 are present around the absorber 10 and the core wraps 20a and 20b.

The magnitude relationship between the absorber 10, the core wraps 20a and 20b, the liquid permeable sheet 30, and the liquid impermeable sheet 40 is not particularly limited, and is appropriately adjusted according to the use of the absorbent article or the like. In addition, the method of retaining the shape of the absorber 10 using the core wraps 20a and 20b is not particularly limited, and as shown in Fig. 1, the absorber may be wrapped by a plurality of core wraps, and the absorber is wrapped by one core wrap.

According to the present embodiment, it is possible to provide a liquid absorbing method using the water-absorbent resin particles, the absorber or the absorbent article of the present embodiment. The liquid absorbing method of the present embodiment includes a step of bringing the liquid to be absorbed into contact with the water-absorbent resin particles, the absorber or the absorbent article of the present embodiment.

**Examples**

Hereinafter, contents of the present invention will be described in further detail using examples and comparative examples, but the present invention is not limited to the following examples.

<Preparation of water-absorbent resin particles>

(Example 1)

A round-bottomed cylindrical separable flask with the inner diameter of 11 cm and the internal volume of 2 L equipped with a reflux cooling device, a dropping funnel, a nitrogen gas introduction tube, and a stirrer (a stirrer blade having two stages of four inclined paddle blades with the blade diameter of 5 cm) was prepared. Into this flask, 293 g of n-heptane was put as a hydrocarbon dispersion medium and 0.736 g of a maleic anhydride-modified ethylene/propylene copolymer (manufactured by Mitsui Chemicals, Inc., High Wax 1105A) was added as a polymeric dispersant to obtain a mixture. The dispersant was dissolved by raising the temperature to 80°C while stirring the mixture, and then the mixture was cooled to 50°C.

Subsequently, 92.0 g of 80.5% by mass aqueous acrylic acid solution (acrylic acid: 1.03 mol) was put into a beaker having the internal volume of 300 mL as a water-soluble ethylenically unsaturated monomer. Subsequently, while cooling from the outside, 147.7 g of 20.9% by mass sodium hydroxide aqueous solution was added dropwise into the beaker to perform 75 mol% of neutralization. Thereafter, 0.092 g of hydroxyethyl cellulose (manufactured by Sumitomo Seika Chemicals Co., Ltd., HEC AW-15F) as a thickener, 0.0736 g (0.272 mmol) of potassium persulfate as a water-soluble radical polymerization initiator, and 0.010 g (0.057 mmol) of ethylene glycol diglycidyl ether as an internal crosslinking agent were added, and then dissolved therein to prepare a first stage aqueous solution.

Then, the above-mentioned first stage aqueous solution was added into the above-mentioned separable flask, and then stirred for 10 minutes. Thereafter, a surfactant solution obtained by heat-dissolving 0.736 g of sucrose stearic acid ester (surfactant, manufactured by Mitsubishi-Chemical Foods Corporation, Ryoto Sugar Ester S-370, HLB: 3) in 6.62 g of n-heptane was added into the separable flask. Then, the inside of the system was sufficiently replaced with nitrogen while stirring at the stirring speed of 550 rpm of the stirrer. Thereafter, the flask was immersed in a water bath at 70°C to raise the temperature, and polymerization was performed for 60 minutes to obtain a first stage polymerization slurry solution.

Subsequently, 128.8 g of 80.5% by mass aqueous acrylic acid solution (acrylic acid: 1.43 mol) was put into another beaker having the internal volume of 500 mL as a water-soluble ethylenically unsaturated monomer. Subsequently, while cooling from the outside, 159.0 g of 27% by mass sodium hydroxide aqueous solution was added dropwise into the beaker to perform 75 mol% of neutralization. Thereafter, 0.090 g (0.334 mmol) of potassium persulfate was added as a water-soluble radical polymerization initiator and then dissolved therein to prepare a second stage aqueous solution.

While stirring at the rotation speed of 1000 rpm of the stirrer, the inside of the above-mentioned separable flask was cooled to 25°C, and then the total amount of the above-mentioned second stage aqueous solution was added to the above-mentioned first stage polymerization slurry solution. Subsequently, after replacing the inside of the system with nitrogen for 30 minutes, the flask was immersed in a water bath at 70°C again to raise the temperature, and the polymerization reaction was performed for 60 minutes. After the polymerization, 0.580 g of 2% by mass ethylene glycol diglycidyl ether aqueous solution (ethylene glycol diglycidyl ether: 0.067 mmol) was added as a crosslinking agent to obtain a second stage hydrogel-like polymer.

To the above-mentioned second stage hydrogel-like polymer, 0.265 g of 45% by mass diethylenetriamine pentasodium pentaacetate aqueous solution was added under stirring. Thereafter, the temperature of the reaction solution was raised in an oil bath at 125°C, and 256.1 g of water was extracted to the outside of the system while refluxing n-heptane by azeotropic distillation of n-heptane and water. Then, 4.42 g of 2% by mass ethylene glycol diglycidyl ether aqueous solution (ethylene glycol diglycidyl ether: 0.507 mmol) was added into the flask as a surface crosslinking agent, and then the mixture was held at 83°C for 2 hours.

Thereafter, n-heptane was evaporated at 125°C and dried to obtain a dried product (polymer particles). This dried product was passed through a sieve having the opening of 850 $\mu$m. Then, 0.2% by mass amorphous silica (Tokusil NP-S manufactured by Oriental Silicas Corporation) was mixed with the dried product based on the total mass of the dried product to obtain 230.8 g of water-absorbent resin particles. The water retention amount (25°C) of physiological saline of the water-absorbent resin particles was 41 g/g.

(Example 2)

The same operation as that in Example 1 was performed except that the amount of water extracted to the outside of the system by azeotropic distillation was changed to 247.9 g after adding a diethylenetriamine pentasodium pentaacetate aqueous solution to the second stage hydrogel-like polymer, and thereby 231.0 g of water-absorbent resin particles were obtained. The water retention amount (25°C) of physiological saline of the water-absorbent resin particles was 35 g/g.

(Example 3)

The same operation as that in Example 1 was performed except that, when preparing the first stage aqueous solution, 0.092 g (0.339 mmol) of 2,2'-azobis (2-amidinopropane) dihydrochloride was added, the use amount of potassium persulfate was changed to 0.018 g (0.068 mmol), the use amount of ethylene glycol diglycidyl ether was changed to 0.005 g (0.029 mmol), when preparing the second stage aqueous solution, 0.129 g (0.475 mmol) of 2,2'-azobis (2-amidinopropane) dihydrochloride was added, the use amount of potassium persulfate was changed to 0.026 g (0.095 mmol), and the amount of water extracted to the outside of the system by azeotropic distillation was changed to 217.8 g after adding a diethylenetriamine pentasodium pentaacetate aqueous solution to the second stage hydrogel-like polymer, and thereby 229.6 g of water-absorbent resin particles were obtained. The water retention amount (25°C) of physiological saline of the water-absorbent resin particles was 44 g/g.

(Comparative Example 1)

The same operation as that in Example 1 was performed except that, when preparing the second stage aqueous solution, 0.0116 g (0.067 mmol) of ethylene glycol diglycidyl ether was added as an internal crosslinking agent in addition to the water-soluble radical polymerization initiator, when obtaining the second stage hydrogel-like polymer, an ethylene glycol diglycidyl ether aqueous solution was not added after performing polymerization reaction for 60 minutes, and the amount of water extracted to the outside of the system by azeotropic distillation was changed to 278.9 g after adding a diethylenetriamine pentasodium pentaacetate aqueous solution to the second stage hydrogel-like polymer, and thereby 230.8 g of water-absorbent resin particles were obtained.

<Evaluation of water retention amount>

Each of an aqueous solution containing 0.01% by mass sodium chloride (NaCl), an aqueous solution containing 0.01% by mass potassium chloride (KCl), an aqueous solution containing 0.01% by mass magnesium chloride ($MgCl_2$), and an aqueous solution containing 0.01% by mass calcium chloride ($CaCl_2$) was prepared. Subsequently, 2.0 g of the above-mentioned water-absorbent resin particles were put into a cotton bag (Membroroad No. 60, 155 mm × 370 mm) installed in a 2 L beaker, and then 2000 mL of each aqueous solution was poured into the cotton bag and at the same time the entire cotton bag was immersed in an aqueous solution. An upper portion of the cotton bag was tied with a rubber band and allowed to stand for 30 minutes to swell the water-absorbent resin particles. After 30 minutes, the cotton bag was dehydrated for 1 minute using a dehydrator (manufactured by KOKUSAN Co., Ltd., product number: H-122) having the centrifugal force set to 167 G. Then, the mass Wa (g) of the cotton bag containing a swollen gel after dehydration was measured. The same operation was performed without adding the water-absorbent resin particles, and an empty mass Wb (g) of the cotton bag when being wet was measured. The water retention amount (water retention amount per unit mass, 25°C) of the water-absorbent resin particles was calculated from the formula below. In addition, a standard deviation between the water retention amounts of the four aqueous solutions was calculated. The results are shown in Table 1.

$$\text{Water retention amount } [g/g] = (Wa-Wb)/2$$

<Preparation of absorber>

Using an air flow type mixer (Padformer, manufactured by O-tec Co., Ltd.), 10 g of the above-mentioned water-absorbent resin particles and 10 g of pulverized pulp were uniformly mixed by air papermaking to prepare a sheet-shaped absorber having the size of 40 cm × 12 cm.

<Evaluation of permeation rate for absorber>

A liquid injection cylinder having the inner diameter of 3 cm was placed in the center of an absorber in a room having the temperature of 25 ± 2°C. Subsequently, 80 mL of a test solution adjusted to 25 ± 1°C was put into the cylinder at one time, a stopwatch was started at the same time, and then an absorption time from the start of putting until the solution was completely absorbed by the absorber was measured. This operation was performed twice more at intervals of 30 minutes (three times in total), and the total value of the absorption time was obtained as the permeation rate (seconds). Using test solutions A and B shown in Table 2 below, the permeation rate of each test solution was evaluated. Blue No. 1 manufactured by Daiwa Kasei Co., Ltd. was used in the two kinds of test solutions. As 1% by mass Triton X solution in the test solution B, a mixture of Triton X-100 manufactured by Wako Pure Chemical Industries, Ltd. and water was used. In addition, the standard deviation between the permeation rates of the two test solutions was calculated. The results are shown in Table 1.

[Table 1]

| | Water retention amount [g/g] | Permeation rate of absorber [second] | | | | | | |
|---|---|---|---|---|---|---|---|---|
| | NaCl | KCl | MgCl$_2$ | CaCl$_2$ | Standard deviation | Test solution | | Standard deviation |
| | | | | | | A | B | |
| Example 1 | 210 | 229 | 229 | 198 | 13 | 133 | 99 | 17 |
| Example 2 | 174 | 180 | 202 | 165 | 14 | 109 | 73 | 18 |
| Example 3 | 267 | 263 | 280 | 249 | 11 | 113 | 89 | 12 |
| Comparative Example 1 | 281 | 288 | 334 | 281 | 22 | 162 | 116 | 23 |

[Table 2]

| Component | Composition (% by mass) | |
|---|---|---|
| | A | B |
| NaCl | 0.795 | 1.000 |
| K$_2$SO$_4$ | 0.197 | - |
| MgSO$_4$ | 0.110 | - |
| MgCl$_2$ | - | 0.281 |
| CaCl$_2$ | 0.062 | 0.027 |
| (NH$_2$)$_2$CO | 1.940 | - |
| 1% by mass Triton X solution | - | 0.250 |
| Blue No. 1 | 0.0025 | 0.0025 |
| Water | Residue | Residue |

According to Table 1, it was confirmed that obtaining a suitable standard deviation in relation to a water retention amount of an aqueous solution of a specific metal salt in the water-absorbent resin particles is effective to obtain an absorber capable of stably obtaining a suitable permeation rate without depending on the composition of the liquid.

**Reference Signs List**

10: absorber, 10a: water-absorbent resin particles, 10b: fiber layer, 20a, 20b: core wrap, 30: liquid permeable sheet, 40: liquid impermeable sheet, 100: absorbent article.

**Claims**

1.   Water-absorbent resin particles comprising:

a crosslinking polymer comprising a structural unit derived from an ethylenically unsaturated monomer,
wherein the ethylenically unsaturated monomer comprises at least one compound selected from the group consisting of (meth)acrylic acid and a salt thereof,
a ratio of the (meth)acrylic acid and the salt thereof is 70 to 100 mol% with respect to a total amount of monomers for obtaining the water-absorbent resin particles, and
a standard deviation between a water retention amount of 0.01% by mass sodium salt aqueous solution, a water retention amount of 0.01% by mass potassium salt aqueous solution, a water retention amount of 0.01% by mass magnesium salt aqueous solution, and a water retention amount of 0.01% by mass calcium salt aqueous solution is 20 g/g or less.

2. The water-absorbent resin particles according to claim 1,
   wherein at least one selected from the group consisting of a water retention amount of 0.01% by mass sodium salt aqueous solution, a water retention amount of 0.01% by mass potassium salt aqueous solution, a water retention amount of 0.01% by mass magnesium salt aqueous solution, and a water retention amount of 0.01% by mass calcium salt aqueous solution is 150 g/g or more.

3. An absorber comprising:
   the water-absorbent resin particles according to claim 1 or 2.

4. An absorbent article comprising:
   the absorber according to claim 3.

5. The absorbent article according to claim 4, which is a diaper.

# Fig.1

## INTERNATIONAL SEARCH REPORT

| International application No. |
|---|
| PCT/JP2019/048819 |

**A. CLASSIFICATION OF SUBJECT MATTER**

C08F 20/06(2006.01)i; C08J 3/12(2006.01)i; A61F 13/53(2006.01)i; B01J 20/26(2006.01)i; B01J 20/28(2006.01)i
FI: C08J3/12 Z CEY; A61F13/53 300; B01J20/26 D; B01J20/28 Z; C08F20/06

According to International Patent Classification (IPC) or to both national classification and IPC

**B. FIELDS SEARCHED**

Minimum documentation searched (classification system followed by classification symbols)

C08J3/00-3/28; C08F6/00-246/00,301/00; A61L15/16-15/64; A61F13/15-13/84; B01J20/26

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 3-247293 A (JAPAN SYNTHETIC RUBBER CO., LTD.) 05.11.1991 (1991-11-05) entire text | 1-5 |
| A | JP 2003-88552 A (SUMITOMO SEIKA CHEMICALS CO., LTD.) 25.03.2003 (2003-03-25) entire text | 1-5 |
| A | WO 2018/181565 A1 (SUMITOMO SEIKA CHEMICALS CO., LTD.) 04.10.2018 (2018-10-04) entire text | 1-5 |

☐ Further documents are listed in the continuation of Box C. ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 02 March 2020 (02.03.2020) | 17 March 2020 (17.03.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2019/048819

| Patent Documents referred in the Report | Publication Date | Patent Family | Publication Date |
|---|---|---|---|
| JP 3-247293 A | 05 Nov. 1991 | (Family: none) | |
| JP 2003-88552 A | 25 Mar. 2003 | (Family: none) | |
| WO 2018/181565 A1 | 04 Oct. 2018 | CN 110446726 A KR 10-2019-0127698 A | |

Form PCT/ISA/210 (patent family annex) (January 2015)

**EP 3 896 094 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H6345819 A
- JP H9510889 A
- WO 2018181565 A